# EUROPÄISCHE PATENTANMELDUNG

(11) **EP 2 826 852 A1**
(43) Veröffentlichungstag der Anmeldung: **21.01.2015**
(21) Anmeldenummer: 14177285.5
(22) Anmeldetag: 16.07.2014
(51) Int. Cl.: C12M 1/107, C12M 1/00, C12M 1/26, C12M 1/34, C12M 1/36

(54) **Biogasanlage zur Erzeugung von Biogas aus Biomasse sowie ein Verfahren zu ihrem Betrieb**

(30) Priorität: 19.07.2013 DE 102013107753; 11.04.2014 DE 102014105185
(71) Anmelder: Lutz, Peter, 81925 Unterföhring (DE)
(72) Erfinder: Lutz, Peter, 81925 Unterföhring (DE)
(74) Vertreter: Alber, Norbert

(57) **Zusammenfassung**

Indem zuverlässig verhindert wird, dass der Perkolatspiegel im Fermenter niemals über das Niveau des Bodens des Fermenters, in dem die Biomasse lagert, ansteigt, wird verhindert, dass die im unteren Bereich liegende Biomasse sich auch nur ein einziges Mal mit Perkolat vollsaugen kann. Dies wird unter anderem durch eine Perkolat-Ausgleichsleitung **(25)** zwischen allen Pumpensümpfen erreicht, und gegebenenfalls durch eine separate Gas-Ausgleichsleitung **(26)** zwischen den Gasräumen aller Pumpensümpfe.

## Beschreibung

### I. Anwendungsgebiet

Die Erfindung betrifft eine Biogasanlage zur Erzeugung von Biogas aus Biomasse.

### II. Technischer Hintergrund

Heute wird Biomasse, beispielsweise Silage oder Bioabfälle oder die organische Fraktion des Hausmülls, auf zwei grundsätzlich unterschiedliche Arten anaerob vergoren:
- Einerseits im Nassgär-Verfahren, bei dem die Biomasse in Form einer pumpfähigen Schlempe vorliegt,
- andererseits im hier vorliegenden Feststoff-Verfahren, bei der die Biomasse, nur durch das Animpfen mit bereits vergorenem Material, in die Fermenter eingebracht wird.

Bei diesem Feststoff-Verfahren, dem so genannten Trockenfermentationsverfahren, sammelt sich Sickerflüssigkeit, das so genannte Perkolat, am Boden der Fermenter, welches aus der in der Biomasse enthaltenen Flüssigkeit besteht, die durch das Eigengewicht der Biomasse heraus gepresst wird, und die mit Fortschritt des Umsetzungsprozesses zusätzlich biologisch aktive Gär-Stoffe enthält.

Dabei ist es üblich, dieses Perkolat aus dem Bodenbereich der Fermenter abzupumpen und wieder auf der Oberseite der Biomasse im Fermenter zu versprühen.

Dadurch werden die biologisch aktiven Stoffe im Perkolat gleichmäßig über das gesamte Volumen der Biomasse im Fermenter verteilt, indem das Perkolat flächig von oben nach unten durch die Biomasse hindurchsickert. Gleichzeitig kann eine Temperaturregelung im Fermenter mittels des zirkulierenden Perkolates erreicht werden, indem dieses vor der Rückführung nach Bedarf beheizt wird.

Dabei unterscheidet sich sowohl die Menge als auch eventuell die Inhaltsstoffe des einem Fermenter zuzuführenden Perkolates abhängig davon, in welchem Zustand der Umsetzungsprozesse sich befindet, welche Art von ursprünglicher Biomasse er enthält und aufgrund von weiteren Faktoren.

Die im Bodenbereich des Fermenters hierfür vorgesehene Perkolat-Sammeleinrichtung war dabei bisher manchmal so ausgebildet, dass mit ihrer Hilfe auch ein Aufstauen des Perkolates im Fermenter möglich war, da dies zumindest kurzzeitig eine erhöhte Ausbeute an Biogas bewirkte.

Als nachteilig hat sich dabei jedoch erwiesen, dass hierdurch im unteren Bereich der Biomasse, in dem das Perkolat aufgestaut war, die Biomasse sehr stark durchfeuchtet wurde und auf dem Perkolat aufschwimmt und dadurch nachteilige Ereignisse ausgelöst werden.

Vor allem jedoch müssen die am Ende des anaeroben Gärprozesses vorhandenen Gärreste weiterverarbeitet werden, meist mittels einer aeroben Gärung zu Kompost weiterverarbeitet werden, was einen Feuchtigkeitsgehalt unterhalb eines bestimmten Schwellenwertes erfordert, der dann durch aufwändige Trocknung erreicht werden muss.

Die durch aufgestautes Perkolat im unteren Bereich sehr feuchten Gärreste auf diesen maximal zulässigen Feuchtigkeitswert herunterzutrocknen erfordert einen hohen Aufwand, entweder für eine sehr große Menge hindurchzuführender, warmer Trocknungsluft oder bei noch höherer Feuchtigkeit für eine erste mechanische Entwässerungsstufe, was einen zusätzlichen hohen Investitionsaufwand bedeutet.

### III. Darstellung der Erfindung

### a) Technische Aufgabe

Es ist daher die Aufgabe gemäß der Erfindung, eine Perkolat-Sammeleinrichtung zur Verfügung zu stellen, die zuverlässig ein Aufstauen von Perkolat im Fermenter vermeidet.

### b) Lösung der Aufgabe

Diese Aufgabe wird durch die Merkmale der Ansprüche 1 und 12 gelöst. Vorteilhafte Ausführungsformen ergeben sich aus den Unteransprüchen.

Der Grundgedanke der Erfindung besteht darin, zu verhindern, dass sich die Biomasse unerwünscht mit zu viel Perkolat vollsaugen kann, indem in den einzelnen Fermentern die Höhe des Perkolatspiegels immer, also vorzugsweise unter allen Umständen, unterhalb des Bodens des Fermenters bleibt.

Denn obwohl die Verweildauer der Biomasse im Fermenter viele Wochen beträgt, kann eine einzige Aufstauung an Perkolat bewirken, dass der dabei erreichte Feuchtigkeitswert nur durch energieintensive aktive Maßnahmen gesenkt werden kann, beispielsweise nach dem Öffnen des Fermenters und bei der Weiterverarbeitung der Biomasse.

Wenn bei einer gattungsgemäßen Biogasanlage die Perkolat-Sammelräume der einzelnen Perkolat-Entnahmevorrichtungen durch Perkolat-Ausgleichs-leitungen miteinander verbunden sind, deren Mündungen in den Sammel-räumen alle tiefer liegen als der Boden des diesem Sammelraum zugeordneten Fermenters, so findet ein Niveau-Ausgleich des Perkolat-Spiegels über alle Sammelräume statt.

Dies bewirkt, dass für den Fall, dass eine der in den Sammelräumen vorhandenen Pumpen, mittels denen das Perkolat aus dem Sammelraum in den zentralen Perkolat-Speicher abgepumpt werden soll, nicht abpumpt - beispielsweise weil der entsprechende Füllstandssensor oder die Pumpe selbst defekt ist - durch das Abpumpen einer der anderen Pumpen aus einem der anderen Sammelräume der Perkolatspiegel insgesamt über alle Sammelräume gesenkt wird, also unterhalb des Niveaus des Bodens irgendeines Fermenters gehalten wird.

Dies eröffnet auch die Möglichkeit, nicht alle Sammelräume aller Fermenter mit einer Pumpe zum Abpumpen des Perkolats auszustatten, sondern nur einige der Sammelräume, im Extremfall nur einen einzigen der Sammelräume, denn durch die strömungstechnische Verbindung aller Sammelräume nach dem Prinzip der kommunizierenden Röhren ist es egal, von welcher Stelle aus das Abpumpen von Perkolat durchgeführt wird, um den Perkolatspiegel insgesamt auf der gewünschten Höhe zu halten.

Vorzugsweise werden daher nicht nur einige sondern alle Perkolat-Sammelräume über entsprechende Perkolat-Ausgleichsleitungen miteinander verbunden.

Dennoch soll zusätzlich die Höhe des Perkolatspiegels in den einzelnen Sammelräumen detektiert werden, und insbesondere an eine zentrale Steuerung gemeldet werden, wofür zumindest in einigen, vorzugsweise in allen der Perkolat-Sammeleinrichtungen, insbesondere den Perkolat-Sammelräumen, ein Füllstandssensor vorhanden ist. Üblicherweise wird hierfür ein Drucksensor verwendet, der dann möglichst tief im Sammelraum verbaut wird und die mit steigender Füllstandshöhe höher werdende Flüssigkeitssäule, die auf ihm lastet, misst.

Die Perkolat-Entnahmevorrichtungen in den einzelnen Fermentern umfassen in der Regel eine Sammelrinne, in der sich das Perkolat sammelt und von der aus es in den jeweiligen Sammelraum abfließen kann.

Die Mündungen der Perkolat-Ausgleichsleitungen sollten dabei unterhalb der Höhe der Perkolat-Entnahmevorrichtung, also insbesondere unterhalb des Bodens der Perkolat-Sammelrinne in dem jeweiligen Sammelraum münden, damit kein Perkolat von einem Sammelraum in die Sammelrinne eines anderen Fermenters fließen kann.

Vorzugsweise sollten die Mündungen der Ausgleichsleitungen auch in den Sammelräumen jeweils oberhalb der Höhe der Ansaugöffnung der dortigen eventuell installierten Pumpe angeordnet sein, sodass die Mündungen frei liegen, wenn die Pumpe das Perkolat bis herunter zu ihrer Ansaugöffnung aus dem Sammelraum abgepumpt hat.

Damit dies in allen Sammelräumen auf das gleiche Niveau erfolgt, sollten die Ansaugöffnungen der Pumpen möglichst alle auf gleicher Höhe montiert sein.

Vorzugsweise enthält jede Perkolat-Sammeleinrichtung ein Ventil in der Absaugleitung sowie eine das Ventil und/oder die Pumpe ansteuernde Steuerung.

Ferner ist vorzugsweise ein Rückschlagventil vorhanden, welches das Zurückfließen von Perkolat über die Perkolat-Entnahmevorrichtung in den Fermenter verhindert.

Die Perkolat-Entnahmevorrichtung und auch die evtl. vorhandene Perkolat-Sammelrinne liegen in aller Regel mit ihrer Oberkante auf Höhe oder unterhalb des Bodens des Fermenters. Dementsprechend liegen die Ansaugöffnungen der Pumpen ebenfalls unterhalb des Niveaus des Bodens des Fermenters, um ein Ansteigen des angesammelten Perkolats bis auf diese Höhe zu verhindern.

Die Perkolat-Sammelräume sollten zusätzlich auch über Gas-Ausgleichs-leitungen miteinander verbunden sein, die die Gasräume oberhalb des Perkolatspiegels in den Sammelräumen miteinander verbinden. Dadurch soll sichergestellt werden, dass sich nicht in einem der Sammelräume ein Druck auch im Gasraum aufbaut, der das Perkolat vom Sammelraum zurück in die Sammeleinrichtung und von dort in den Fermenter hochdrückt und die Biomasse durchfeuchtet.

Wenn es sich dabei um separate, also von den Perkolat-Ausgleichsleitungen getrennte, Gas-Ausgleichsleitungen handelt, müssen sich deren Mündungen in den Gasräumen der Sammelräume natürlich möglichst hochliegend befinden, sodass sie vorzugsweise unter allen Umständen oberhalb des Perkolatspiegels des Sammelraumes liegen.

Die Gas-Ausgleichsleitungen müssen auch eine Verbindung zu der Biogas-Entnahmeleitung besitzen, indem sie vorzugsweise mit einer der Biogas-Entnahmeöffnungen eines der Fermenter verbunden sind.

Anstelle separater Gas-Ausgleichsleitungen können hierfür jedoch auch die Perkolat-Ausgleichsleitungen benutzt werden, in denen sie mit den Gas-Ausgleichsleitungen funktionsvereinigt sind.

Hierfür muss jedoch sichergestellt werden, dass diese Ausgleichsleitungen niemals an irgendeiner Stelle über die volle Höhe ihres Querschnittes mit Perkolat gefüllt sind, da dann die Ausgleichsleitungen nicht mehr gleichzeitig einen Gasaustausch zwischen den Gasräumen der einzelnen Sammelräume bewirken könnten.

Hierfür ist es notwendig, dass an keiner Stelle aller Perkolat-Ausgleichs-leitungen der höchste Punkt eines Querschnittes tiefer liegt als der tiefste Punkt eines Querschnittes an irgendeiner anderen Stelle der Perkolat-Ausgleichsleitungen. Denn wenn dies der Fall wäre, würde der Effekt eines Siphons erfolgen, welches einen Gasaustausch von einer zur anderen Seite der mit Flüssigkeit verschlossenen Stelle verhindert.

Ebenso wäre es sinnvoll, wenn keine der Perkolat-Ausgleichsleitungen in ihrem Verlauf einen tiefsten Punkt ihres Querschnittes aufweist, der höher liegt als der höchste Punkt irgendeines Querschnittes an irgendeiner anderen Stelle der Perkolat-Ausgleichsleitungen. Denn dies würde ebenfalls bewirken, dass an der Stelle des tiefstliegenden Querschnittes der Querschnitt vollständig mit Perkolat angefüllt wäre und über diese Stelle hinweg kein Gas-Austausch mehr stattfinden würde.

Als Vorgehensweise sollten die Perkolat-Sammelräume der einzelnen Fermenter strömungstechnisch spätestens dann miteinander verbunden werden, wenn der Perkolatspiegel in einem der Sammelräume über ein vorgegebenes Niveau ansteigt. Dadurch kann das Perkolat von einer beliebigen Stelle der miteinander verbundenen Sammelräume in einen Perkolat-Speicher abgepumpt werden.

Hierzu bedarf es nicht in jedem Sammelraum einer Pumpe und. es ist unschädlich, wenn eine der mehreren vorhandenen Pumpen nicht funktioniert.

Vorzugsweise sind auch die Gasräume oberhalb des Perkolatspiegels der einzelnen Sammelräume ständig in strömungstechnischer Verbindung miteinander gehalten, und diese miteinander verbundenen Gasräume werden vorzugsweise ständig in Verbindung mit wenigstens einer Biogas-Entnahmeöffnung eines der Fermenters gehalten.

Dadurch ist sichergestellt, dass durch den Druckausgleich der Gasräume untereinander und das Absaugen von Biogas über eine der Biogas-Entnahmeöffnungen in keinem der Gasräume ein Überdruck entstehen kann, der im dortigen Sammelraum das Perkolat hoch in den Fermenter drücken könnte.

Diese Maßnahmen sind jedoch lediglich als Notmaßnahmen gedacht, dass der normale Abpumpvorgang aus den Sammelräumen der Perkolat-Entnahmeeinrichtungen nicht funktioniert.

Dieser läuft ja vorzugsweise so ab, dass bei einem Ansteigen von Perkolat über eine erste Stauhöhe des Sammelraumes, die insbesondere auf der Höhe des Bodens des Fermenters liegt, sofort die dortige Pumpe in Gang gesetzt wird und andererseits die Verteilung von Perkolat in diesen Fermenter gestoppt wird, bis der Perkolatspiegel wieder unterhalb der ersten Stauhöhe liegt.

Vorzugsweise wird unterhalb der ersten Stauhöhe eine zweite Stauhöhe ständig überwacht, und bei Erreichen dieser zweiten Stauhöhe bereits - unter Berücksichtigung der Durchsickerungszeit - rechtzeitig das Ausbringen von Perkolat auf der Oberseite dieses Fermenters vermindert oder gestoppt wird, sodass das Perkolat gar nicht weiter bis zur ersten Stauhöhe aufsteigen kann.

Ferner wird vorzugsweise die in einen Fermenter eingebrachte und entnommene Menge an Perkolat über die Menge pro Zeiteinheit gemessen und an die Steuerung weitergegeben, sodass diese in Abhängigkeit davon die weitere zugeführte Menge an Perkolat in diesem Fermenter so steuert, dass auch unter Berücksichtigung der Leistung der Pumpe in diesem Fermenter kein Aufstauen an Perkolat bis zur Höhe des Bodens des Fermenters auftreten kann.

### c) Ausführungsbeispiele

Ausführungsformen gemäß der Erfindung sind im Folgenden beispielhaft näher beschrieben. Es zeigen:
**Fig. 1****:** eine Biogas-Anlage mit Perkolat-Kreislauf,
**Fig. 2** eine Detailvergrößerung eines Pumpensumpfes aus Figur **1****,**
**Fig. 3a****:** zwei miteinander verbundene Pumpensümpfe,
**Fig. 3b****:** drei miteinander verbundene Pumpensümpfe.

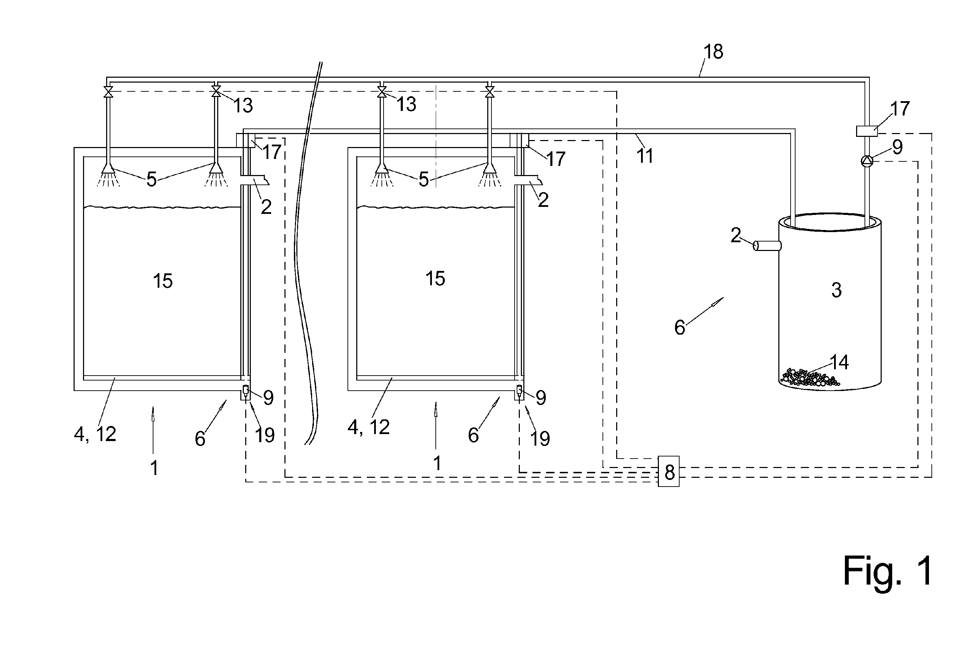

**Figur 1** zeigt eine Biogas-Anlage nach dem Trockenfermentationsprinzip mit dem Perkolat-Kreislauf:

Die Biomasse 15 ist in mehreren parallel betriebenen Fermentern 1 luft- und gasdicht aufgenommen, in denen sie anaerob vergärt. Das entstehende Biogas wird über die Biogas-Entnahmeöffnung **2** aus jedem Fermenter **1** abgezogen und der Verwertung zugeführt.

Am Boden **1a** jedes Fermenters **1** sammelt sich dabei das sog. Perkolat, eine Sickerflüssigkeit, entstehend aus der in der Biomasse **15** enthaltenen Feuchtigkeit, die allein schon durch das Eigengewicht der aufgeschütteten Biomasse **15** herausgedrückt wird.

Das Perkolat wird durch eine Perkolat-Entnahmevorrichtung **4,** in der Regel eine Sammelrinne **12,** die sich im Boden **1a** jedes Fermenters **1** befindet, gesammelt und einer Perkolat-Sammelvorrichtung **6** zugeführt, die auch einen Perkolat-Speicher **3** umfasst.

Aus diesem Perkolat-Speicher **3** wird das Perkolat über eine Perkolat-Verteilvorrichtung **5** in den einzelnen Fermentern **1** auf der Oberseite der Biomasse **15** verteilt ausgebracht, da das Perkolat mit zunehmendem Fortschritt des Umsetzungsprozesses der Biomasse in den Fermentern **1** bioaktive Gärstoffe enthält, die den Umsetzungsprozess fördern.

Aus diesem Grund können im Perkolat-Speicher **3** auch Füllkörper **14** vorhanden sein, an denen sich die bioaktiven Mikroorganismen gut anlagern können, und die somit zum einen ein Reservoir an bioaktiven Gärstoffen für das Perkolat bilden und andererseits bewirken, dass im Perkolat-Speicher **3** selbst ebenfalls Biogas entsteht, welches über eine Biogas-Entnahmeöffnung **2** entnommen und genutzt werden kann.

Zu diesem Zweck ist in jeder der Stichleitungen der Perkolat-Verteilvorrichtung 5 in die Fermenter **1** hinein jeweils ein Ventil **13** angeordnet, welches nur bei Perkolat-Zufuhr in diese Stichleitung hinein geöffnet wird.

In der Regel ist immer nur eine der Stichleitungen der gesamten Biogas-Anlage geöffnet, sodass ein einziger Durchflussmesser **17** zum Messen der Perkolatmenge in der Perkolat-Versorgungsleitung **18** ausreicht, um die an jeder Stichleitung ausgebrachte Menge an Perkolat zu erfassen. Damit ist die insgesamt und auch pro Zeiteinheit in jedem der Fermenter ausgebrachte Perkolatmenge bekannt, und kann ebenfalls der Steuerung **8** zur Verfügung gestellt werden.

Von der Perkolat-Entnahmevorrichtung **4,** in der Regel eine Sammelrinne **12,** jedes Fermenters führt eine Verbindungsleitung zu einer Perkolat-Sammelleitung **11,** die im Perkolatbehälter **3** endet.

Um erfindungsgemäß das Aufstauen von angefallenem Perkolat im Fermenter **1** über das Niveau des Bodens **1a** des Fermenters **1** hinaus zu vermeiden, ist in jeder der Verbindungen von der Perkolat-Entnahmevorrichtung **4** jedes Fermenters **1** zur Perkolat-Sammelleitung **11** zumindest eine ansteuerbare Pumpe **9** vorhanden, um das in der Perkolat-Entnahmevorrichtung **4** angefallene Perkolat abzupumpen, bevor es sich über die Oberkante des Bodens **1a** des Fermenters **1** aufstaut.

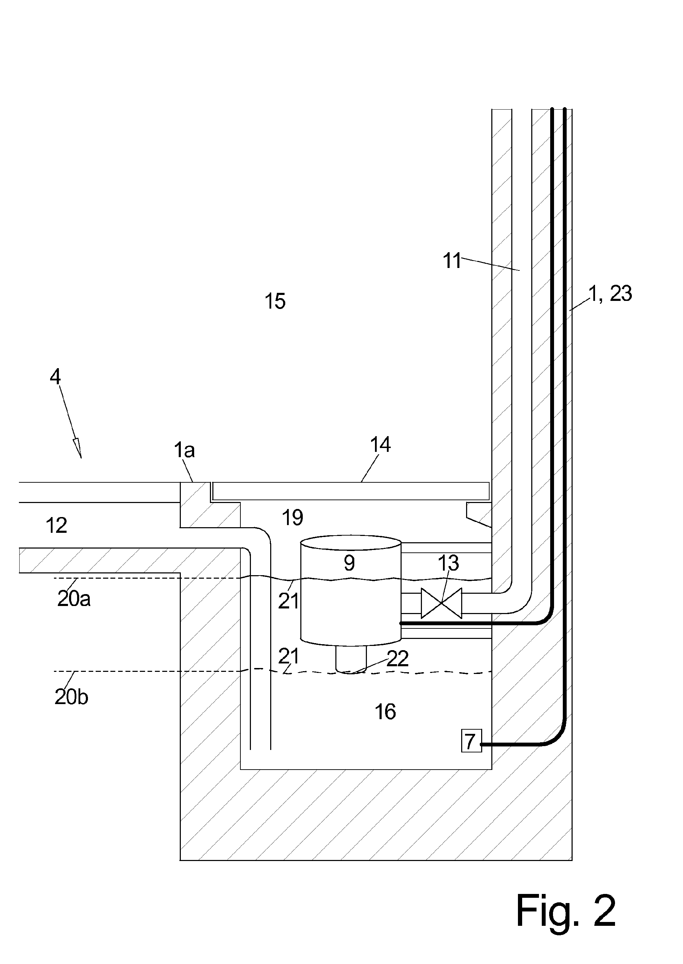

In der Regel ist die Sammelrinne **12** der Perkolat-Entnahmevorrichtung **4** mit der Oberkante bündig zur Oberkante des Bodens **1a** des Fermenters eingelassen, damit die Sammelrinne **12** auch problemlos beim Be- und Entladen des Fermenters mit schwerem Gerät überfahren werden kann, wie am besten **Figur 2** zeigt.

Die Pumpe **9** ist dabei in einer Vertiefung im Boden **1**a des Fermenters **1,** dem sogenannten Pumpensumpf **19,** der als Sammelraum **24** für Perkolat dient, montiert, und die tiefer ist als die Sammelrinne **12,** sodass die Pumpe **9** mit ihrer Ansaugöffnung **22** unterhalb der Oberkante des Bodens **1a** und sogar unterhalb der Höhe der Unterkante der Sammelrinne **12** positioniert ist. Die Ablaufleitung für das Perkolat aus der Sammelrinne **12** - die hierfür ein Gefälle zur Ablaufleitung hin aufweist - endet frei im Pumpensumpf **19,** jedoch kann darin ein Rückschlagventil **10** angeordnet sein, um ein Zurückdrücken von Perkolat zu verhindern.. Dadurch stellt sich im Pumpensumpf **19** immer ein Perkolatspiegel **21** auf Niveau oder oberhalb der Ansaugöffnung **22** der Pumpe **9** ein.

Die Pumpe **9** fördert das von ihr angesaugte Perkolat **16** über eine in der Wand des Fermenters **1** nach oben verlaufende Steigleitung zur Perkolat-Sammelleitung **11.**

Ein Drucksensor **7** ist als Füllstandssensor an einer möglichst tiefliegenden Stelle des Pumpensumpfes **19** montiert und zeigt die momentane Stauhöhe an Perkolat im Pumpensumpf an. z.B. eine erste Stauhöhe **20**a und eine zweite Stauhöhe **20**b**.** Bei deren Erreichen können die beschriebenen Reaktionen seitens der Steuerung **8** erfolgen.

Zusätzlich ist auch ein Ventil **13,** in der Steigleitung vorhanden, um diese absperren zu können. In der Regel ist das Ventil **13** nur geöffnet, wenn die Pumpe **9** läuft.

Angesteuert wird die Pumpe **9** von einer Steuerung **8,** die mit dem Drucksensor 7 in Verbindung steht.

Der Ansaugpunkt der Pumpe **9** liegt dabei vorzugsweise unterhalb der Höhe der zweiten, unteren Stauhöhe **20**b. Falls das Perkolat bis zur Höhe des oberen, erste Stauhöhe **20**a steht, bewirkt die Steuerung **8**
- entweder, dass die Pumpe **9** in Gang gesetzt wird und das Perkolat abgepumpt wird,
- und/oder die auf der Oberseite der Biomasse **15** in diesem Fermenter **1** ausgebrachte Menge an Perkolat verringert oder auf null gesetzt wird:

Denn das Perkolat benötigt geraume Zeit für die Durchsickerung der mehrere Meter hoch aufgeschütteten Biomasse **15** von oben nach unten, sodass der Mengenanfall an Perkolat am Boden 1a des Fermenters, also in der Sammelrinne **12,** erheblich zeitversetzt zum Ausbringungszeitpunkt auf der Oberseite der Biomasse **15** erfolgt.

Der obere, erste Stauhöhe **20**a soll vom Perkolatspiegel nach Möglichkeit gar nie erreicht werden, sondern dient lediglich der Sicherheit, dass eine zweite Überwachungsstufe existiert: Denn wenn dieser obere, erste Stauhöhe **20**a erreicht ist, wird daraufhin die Pumpe **9** in Gang gesetzt und läuft solange, bis die obere, erste Stauhöhe **20**a unterschritten ist und insbesondere auch die untere, zweite Stauhöhe **20**b unterschritten ist.

Es versteht sich von selbst, dass die Leistung der Pumpe **9** ausreichend sein muss, um auch den größtmöglichen Anfall an Perkolat in der Perkolat-Entnahmevorrichtung **4** bewältigen zu können, also den Perkolatspiegel in der Perkolat-Entnahmevorrichtung **4** absenken zu können.

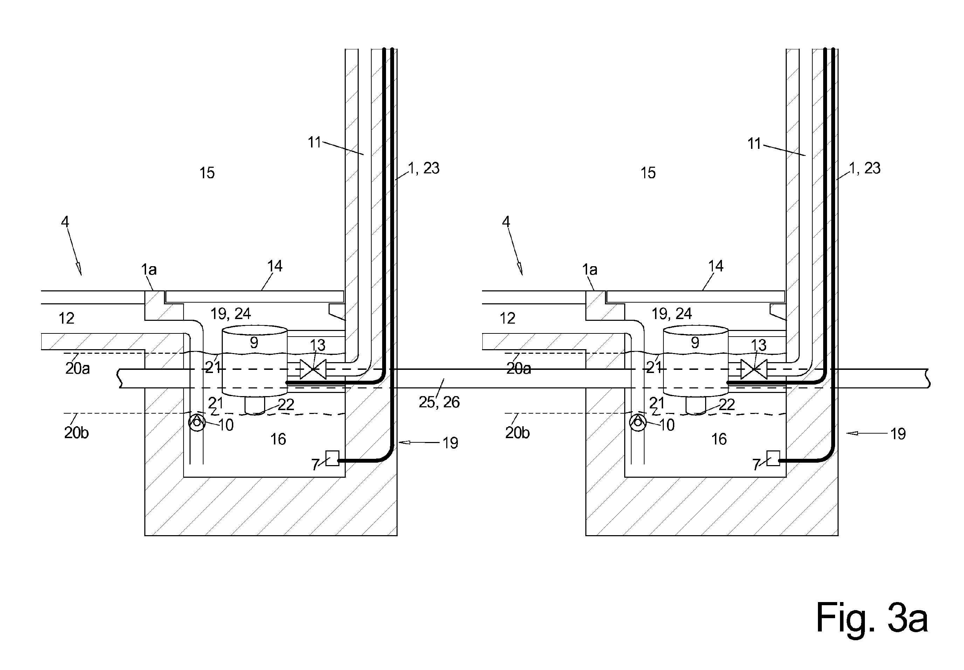

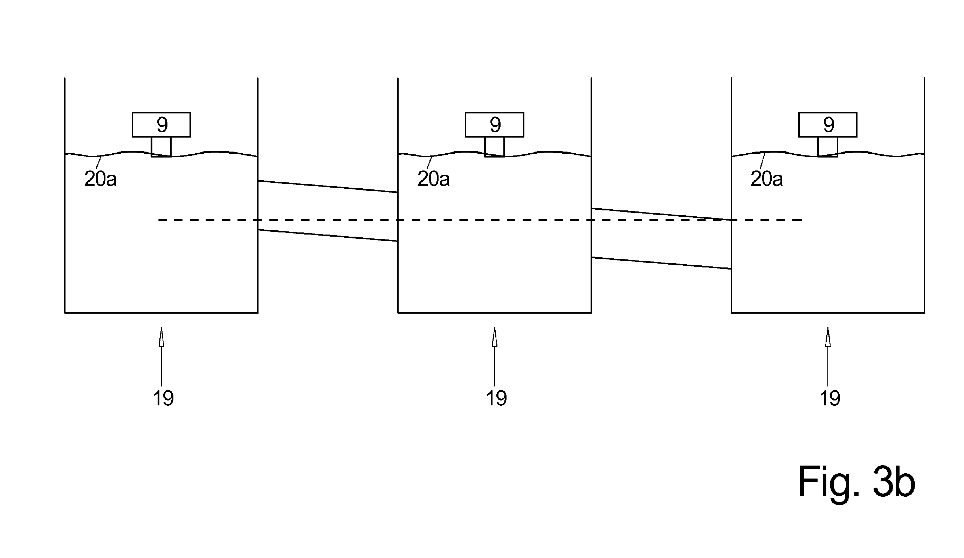

Die **Figuren 3a** und **3b** zeigen zusätzliche Sicherungsmaßnahmen, um das Ansteigen des Perkolats bis zur Höhe des Bodens **1a** des Fermenters 1 unter allen Umständen zu vermeiden:
Wie in **Figur 3a** auszugsweise dargestellt, sind alle Perkolat-Sammelräume **24,** also alle Pumpensümpfe **19,** der Fermenter 1 über eine Perkolat-Ausgleichsleitung **25** miteinander verbunden, die dadurch gleichzeitig als Gas-Ausgleichsleitung **26** fungiert, indem sie gerade und horizontal die einzelnen Sammelräume **24** verbindet, also in ihrem Verlauf auch keine Durchbiegungen nach unten aufweist.

Denn wie die Prinzipskizze der **Figur 3b** zeigt, ist bei einer ausreichenden Horizontallage, so dass an keiner Stelle der tiefste Punkt der Ausgleichsleitung **25** höher liegt als der niedrigste der höchsten Punkte der Ausgleichsleitungen **25,** sichergestellt, dass an keiner Stelle der Querschnitt der Ausgleichsleitungen **25, 26** vollständig mit Flüssigkeit, also Perkolat, angefüllt ist, also der obere Teil des Querschnittes frei ist für die Durchströmung von Gas und damit den Ausgleich von Gas zwischen den Gasräumen oberhalb des Perkolatspiegels der einzelnen Perkolat-Sammelräume **24.**

Diese Ausgleichsleitung **25** stellt zum einen sicher, dass bei einem Abpumpen auch nur in einem einzigen Sammelraum **24** mittels der dortigen Pumpe **9** der Perkolatspiegel **21** in allen Sammelräumen **24** dadurch gesenkt werden kann. Da die Ausgleichsleitung **25, 26** an einer Stelle auch mit der Biogas-Entnahme-Leitung, also beispielsweise einer der Biogas-Entnahmeöffnungen **2**eines der Fermenter, in Verbindung steht, wird auch aus den miteinander verbundenen Gasräumen ständig das in den Sammelräumen **24** entstehende Biogas abgesaugt und es kann in keinem der Gasräume kein Überdruck entstehen, der Perkolat in den dortigen Fermenter **1** hochdrücken könnte.

### BEZUGSZEICHENLISTE

- 1: Fermenter
- **1**a: Boden
- **2**: Biogas-Entnahmeöffnung
- **3**: Perkolat-Speicher
- **4**: Perkolat-Entnahmevorrichtung
- **5**: Perkolat-Verteilvorrichtung
- **6**: Perkolat-Sammeleinrichtung
- **7**: Füllstandssensor
- **8**: Steuerung
- **9**: Pumpe
- **10**: Rückschlagventil
- **11**: Perkolat-Sammelleitung
- **12**: Sammelrinne
- **13**: Ventil
- **14**: Füllkörper
- **15**: Biomasse
- **16**: Perkolat
- **17**: Durchflusssensor
- **18**: Perkolat-Versorgungsleitung
- **19**: Pumpensumpf
- **20a**: erste Stauhöhe
- **20b**: zweite Stauhöhe
- **21**: Perkolat-Spiegel
- **22**: Ansaugöffnung
- **23**: Wand
- **24**: Sammelraum
- **25**: Perkolat-Ausgleichsleitung
- **25a, b**: Mündung
- **26**: Gas-Ausgleichsleitung

## Patentansprüche

1. **Biogasanlage** zur Methanisierung von nicht-pumpbarer Biomasse, mit
- mehreren gas- und flüssigkeitsdicht verschließbaren Fermentern **(1)** für die Biomasse,
- einer Biogas-Entnahmeöffnung **(2)** in jedem Fermenter **(1),**
- einer Perkolat-Sammeleinrichtung **(6)** mit insbesondere einem Perkolat-Speicher **(3)** und
- einer Perkolat-Entnahmevorrichtung **(4)** in jedem Fermenter **(1),** die das anfallende Perkolat zu der Perkolat-Sammeleinrichtung **(6)** befördert und einen Perkolat-Sammelraum **(24)** in einer Höhe unterhalb des Bodens **(1**a**)** des Fermenters **(1)** aufweist,
- einer Perkolat-Verteilvorrichtung **(5),** die das Perkolat aus der Perkolat-Sammeleinrichtung **(6)** in jeden der Fermenter **(1)** ausbringen kann,
**dadurch gekennzeichnet, dass**
die Perkolat-Sammelräume **(24)** durch Perkolat-Ausgleichsleitungen **(25)** miteinander verbunden sind, deren Mündungen (**25a,** b) in den Sammelräumen **(24)** alle tiefer als der Boden (1a) des zugeordneten Fermenters **(1)** liegen.

2. Biogasanlage nach Anspruch **1,**
**dadurch gekennzeichnet, dass**
alle Perkolat-Sammelräume **(24)** durch Perkolat-Ausgleichsleitungen **(25)** miteinander verbunden sind.

3. Biogasanlage nach einem der vorhergehenden Ansprüche,
**dadurch gekennzeichnet, dass**
die Perkolat-Sammeleinrichtung **(6)** wenigstens einen Füllstandsensor **(7),** insbesondere in Form eines Drucksensors **(7),** umfasst, der in einer Höhe unterhalb der Oberkante des Bodens **(1**a**)** des Fermenters **(1)** angeordnet ist.

4. Biogasanlage nach einem der vorhergehenden Ansprüche,
**dadurch gekennzeichnet, dass**
die Perkolat-Entnahmevorrichtung **(4)** eine Sammelrinne **(12)** umfasst.

5. Biogasanlage nach einem der vorhergehenden Ansprüche,
**dadurch gekennzeichnet, dass**
- die Mündungen (**25**a**,** b) unterhalb der Höhe der PerkolatEntnahmevorrichtung **(4),** insbesondere des Bodens der PerkolatSammelrinne **(4),** in dem jeweiligen Sammelraum **(24)** angeordnet sind, und/oder
- die Mündungen (**25a,** b) oberhalb der Höhe der Ansaugöffnung **(22)** der Pumpe **(9)** in dem jeweiligen Sammelraum **(24)** angeordnet sind.

6. Biogasanlage nach einem der vorhergehenden Ansprüche,
**dadurch gekennzeichnet, dass**
die Perkolat-Ausgleichsleitungen (25) in der Seitenansicht betrachtet gerade verlaufen und insbesondere im Wesentlichen horizontal verlaufen

7. Biogasanlage nach einem der vorhergehenden Ansprüche,
**dadurch gekennzeichnet, dass**
- die Ansaugöffnungen **(22)** der Pumpen **(9)** alle auf gleicher Höhe montiert sind, und/oder
- die Perkolat-Sammeleinrichtung **(6)** eine Pumpe **(9)** und/oder ein Ventil **(13),** sowie eine die Pumpe **(9)** und/oder das Ventil **(13)** ansteuernde Steuerung (8) umfasst.

8. Biogasanlage nach einem der vorhergehenden Ansprüche,
**dadurch gekennzeichnet, dass**
die Perkolat-Entnahmevorrichtung **(4)** ein Rückschlagventil **(10)** umfasst, die ein Zurückfließen von Perkolat über die Perkolat-Entnahmevorrichtung **(4)** in den Fermenter **(1)** verhindert.

9. Biogasanlage nach einem der vorhergehenden Ansprüche,
**dadurch gekennzeichnet, dass**
- die Perkolat-Entnahmevorrichtung **(4)** mit ihrer Oberkante mit dem Boden des Fermenters **(1)** fluchtet oder unter diesem liegt, und/oder
- die Ansaugöffnung **(22)** der Pumpe **(9)** unterhalb des Niveaus des Bodens des Fermenters **(1)** angeordnet ist.

10. Biogasanlage nach einem der vorhergehenden Ansprüche,
**dadurch gekennzeichnet, dass**
- entweder an keiner Stelle aller Perkolat- Ausgleichsleitungen **(25)** der höchste Punkt eines Querschnittes tiefer liegt als der tiefste Punkt des Querschnittes an irgendeiner Stelle einer der PerkolatAusgleichsleitungen **(25)**
- oder alle Perkolat-Sammelräume **(24)** über separate Gas-Ausgleichsleitungen **(26)** miteinander verbunden sind, deren Mündungen (**26**a**,** b) höher liegen als die am höchsten gelegenen Mündung **(25a,** b) der Perkolat-Ausgleichsleitungen **(25)** in irgendeinem Perkolat-Speicher **(3).**

11. Biogasanlage nach einem der vorhergehenden Ansprüche,
**dadurch gekennzeichnet, dass**
keine der Perkolat-Ausgleichsleitungen **(25)** in ihrem Verlauf einen tiefsten Punkt ihres Querschnittes aufweist, der höher liegt als der höchste Punkt irgendeines Querschnittes in irgendeiner der Perkolat-Ausgleichsleitungen **(25).**

12. **Verfahren** zur Erzeugung von Biogas aus nicht-pumpbarer Biomasse mit mehreren gas- und flüssigkeitsdicht verschließbaren Fermentern **(1)** für die Biomasse wobei
- das in den Fermentern **(1)** anfallende Perkolat gesammelt wird und
- in den einzelnen Fermentern **(1)** gesteuert wieder ausgebracht wird, **dadurch gekennzeichnet, dass**
die Höhe des Perkolat-Spiegels **(21)** in den Fermentern **(1)** unterhalb des Bodens **(1a)** des Fermenters **(1)** gehalten wird, indem die unterhalb des Bodens **(1a)** der Fermenter **(1)** jeweils angeordneten Perkolat-Sammelräume **(24)** automatisch strömungstechnisch miteinander verbunden werden, spätestens wenn der Perkolatspiegel in einem der Sammelräume **(24)** über ein vorgegebenes Niveau ansteigt und aus mehreren, insbesondere allen, Perkolat-Sammelräumen **(24)** das Perkolats in einen Perkolat-Speicher **(3)** abgepumpt werden kann.

13. Verfahren nach Anspruch **12,**
**dadurch gekennzeichnet, dass**
- die Gasräume oberhalb des Perkolatspiegels **(21)** in den Sammelräumen **(24)** ständig in strömungstechnischer Verbindung miteinander gehalten werden, und insbesondere ständig in strömungstechnischer Verbindung mit wenigstens einer der Biogas-Entnahmeöffnungen **(2)** eines Fermenters **(1)** gehalten wird, und/oder
- bei auftretendem Rückstau von Perkolat über eine erste Stauhöhe **(20**a**),** insbesondere die Höhe des Bodens **(1**a**)** des Fermenters **(1),** hinaus sofort die Pumpe **(9)** in Gang gesetzt wird und die Verteilung von Perkolat auf der Oberseite der Biomasse **(15)** im Fermenter **(1)** gestoppt wird, bis der Perkolat-Spiegel **(21)** unterhalb der ersten Stauhöhe **(20**a**),** insbesondere der Höhe des Bodens **(1**a**)** des Fermenters **(1),** liegt.

14. Verfahren nach einem der vorhergehenden Verfahrensansprüche,
**dadurch gekennzeichnet, dass**
im Höhenbereich unterhalb des Bodens **(1**a**)** des Fermenters **(1)** und insbesondere unterhalb der ersten Stauhöhe **(20**a**),** eine zweite Stauhöhe **(20**b**)** des Perkolatspiegels ständig überwacht wird, und bei Erreichen dieser zweiten Stauhöhe **(20**b**),** unter Berücksichtigung der Durchsickerungszeit, rechtzeitig das Ausbringen von Perkolat auf der Oberseite der Biomasse **(15)** in diesem Fermenter **(1)** vermindert oder gestoppt wird, so dass kein Aufstauen von Perkolat über das Niveau des Bodens **(1**a**)** des Fermenters **(1)** erfolgen kann.

15. Verfahren nach einem der vorhergehenden Verfahrensansprüche,
**dadurch gekennzeichnet, dass**
die in einen Fermenter (1) eingebrachte und entnommene Menge an Perkolat, insbesondere die Menge pro Zeiteinheit, gemessen wird und an die Steuerung **(8)** weitergegeben wird und die weitere zugeführte Menge an Perkolat in diesen Fermenter **(1)** so gesteuert wird, dass unter Berücksichtigung der Leistung der Pumpe **(9)** kein Aufstauen an Perkolat über die Höhe des Bodens **(1**a**)** des Fermenters **(1)** auftreten kann.
